# EUROPÄISCHE PATENTANMELDUNG

(11) **EP 4 335 401 A1**
(43) Veröffentlichungstag der Anmeldung: **13.03.2024**
(21) Anmeldenummer: 22194389.7
(22) Anmeldetag: 07.09.2022
(51) Int. Cl.: A61B 34/10, A61B 18/04, A61B 34/20, A61B 18/00, A61B 90/00, A61B 90/50, A61B 1/303

(54) **BEHANDLUNGSEINRICHTUNG ZUR ERSTELLUNG EINER BEHANDLUNGSPLANKARTE**

(71) Anmelder: Erbe Elektromedizin GmbH, 72072 Tübingen (DE)
(72) Erfinder: Daniel, Yannick, 72336 Balingen (DE); Jurjut, Ovidiu, 72070 Tuebingen (DE); Enderle, Markus, 72070 Tuebingen (DE); Leonberger, Elena, 72793 Pfullingen (DE)
(74) Vertreter: Rüger Abel Patentanwälte PartGmbB

(57) **Zusammenfassung**

Die erfindungsgemäße Behandlungseinrichtung ermöglicht eine Behandlung von Gewebe mit einem keine unmittelbare Gewebespur hinterlassenden Behandlungsmedium (23) sowohl mit manueller als auch mit maschineller Instrumentenführung und unter Anpassung der Behandlungsplankarte aufgrund vorheriger Behandlungen. Die Behandlungsplankarte stellt die Behandlungsvorschrift für den Behandler dar. Das erfindungsgemäße Konzept gestattet die Anpassung an verschiedene Patienten oder Patientenkollektive sowie auch die Individualisierung der Erstellung der Behandlungsplankarte hinsichtlich der nutzenden Behandler.

## Beschreibung

Die Erfindung betrifft eine Behandlungseinrichtung zur Behandlung von menschlichem oder tierischem Gewebe, insbesondere zur Behandlung von Gewebeoberflächen, z.B. zervikalen intraepithelialen Neoplasien. Weiter betrifft die Erfindung ein Verfahren zur Erstellung einer Behandlungsplankarte zur Behandlung von menschlichem oder tierischem Gewebe, insbesondere zur Behandlung von zervikalen intraepithelialen Neoplasien.

Aus der US 2019/036617 A1 ist ein System zur Behandlung von äußeren Hautpartien eines Patienten bekannt. Dazu ist eine Kontrolleinheit mit einer Datenbank verbunden, in welcher Gewebemerkmale und zugehörige Energieparameter abgespeichert sind. Auf der Basis dieser abgespeicherten Daten wird die Energiedosis zur Behandlung der Haut festgelegt. Außerdem kann die Kontrolleinheit patientenindividuelle Parameter wie Alter oder Hauttyp zur Festlegung der Dosierung berücksichtigen. Nach Abgabe der festgelegten Energiedosis wird der Erfolg der Behandlung durch Überprüfung bestimmter Hautmerkmale evaluiert. Gegebenenfalls wird die Behandlung mit einer angepassten Energiedosis wiederholt. Die Kontrolleinheit kann dazu ein Modul zum maschinellen Lernen enthalten.

Weiter ist aus der EP 3 576 100 A1 ein System bekannt, das der Entscheidungsunterstützung bei einer medizinischen Therapie dient. Dazu ist ein Generator vorgesehen, der mittels maschinellen Lernens eine Ergebnisvorhersage einer auf einen Patienten zugeschnittenen Therapie liefert.

Die automatische Therapieplanung in der Radiologie ist zudem in der EP 3 384 962 A1 beschrieben. Zu weiterführendem Stand der Technik wird auf die US2012/283574 A1, die EP 1 644 867 B1, die US 2021/0042915 A1 und die EP 2 237 190 A2 verwiesen.

Zur Behandlung von Gewebe, insbesondere Gewebeoberflächen, kann Plasma angewendet werden, das infolge seiner niedrigen Temperatur und/oder Einwirkungsdauer das Gewebe thermisch nicht bzw. nicht sichtbar schädigt. Für den Behandler ist es dabei kritisch zu erkennen, an welcher Stelle und mit welcher Intensität das Gewebe behandelt werden muss, weil der Therapieerfolg nicht mit bloßem Auge (makroskopisch) wie sonst üblich erkennbar ist. Beispielsweise können zervikale intraepitheliale Neoplasien auf diese Weise behandelt werden.

Zervikale intraepitheliale Neoplasien werden typischerweise in einem Anfärbeversuch identifiziert, bei dem der zu untersuchende Gewebebereich mit Essigsäurelösung und anschließend mit Iod-Kaliumiodid-Lösung behandelt wird, was zu einer allmählich abklingenden essigweißen bzw. jodnegativen Verfärbung des Gewebes führt. Die spezifische Reaktion auf die Färbung markiert pathologische Gewebepartien. Hat der Behandler auf diese Weise erkannt wo er behandeln muss, führt er die Behandlung dementsprechend mehr oder weniger passend durch. Deswegen hängt das Behandlungsergebnis stark von dem Können und der Erfahrung des Behandlers ab. Es können sich sowohl überbehandelte Bereiche als auch unterbehandelte bzw. unregelmäßig/nicht homogen behandelte Bereiche ergeben.

Neben der Essigsäure-Iod-Färbetechnik können auch andere Anfärbetechniken sowie weitere Verfahren genutzt werden, um behandlungswürdiges und/oder pathologisches Gewebe kenntlich zu machen oder zu identifizieren. Beispielsweise sind optische Techniken, wie optische Emissionsspektroskopie (OES), optische Kohärenztomografie (OCT), Raman-Spektroskopie oder hyperspektrale bzw. multispektrale Kameraeinrichtungen einsetzbar. Ebenso kann in manchen Anwendungsfällen darauf vertraut werden, dass das zu behandelnde Gewebe allein auf Grundlage der sichtbaren Unterschiede vom Behandler oder dem medizinischen Kamerasystem erkannt wird. Dies hängt von der Art des zu behandelnden Gewebes sowie der zu behandelnden Gewebeveränderung ab. Zur Erleichterung der optischen Erkennung durch den Behandler können Beleuchtungseinrichtungen mit speziell abgestimmter spektraler Zusammensetzung des zur Beleuchtung des Gewebes verwendeten Lichts eingesetzt werden.

Es ist Aufgabe der Erfindung eine Behandlungseinrichtung sowie ein Verfahren zur Erstellung einer Therapieempfehlung anzugeben, um die Wirksamkeit der Behandlung und die Patientensicherheit zu erhöhen.

Diese Aufgabe wird mit der Behandlungseinrichtung nach Anspruch 1 sowie mit dem Verfahren nach Anspruch 15 gelöst:

Erfindungsgemäß ist eine Behandlungseinrichtung vorgesehen, die eine Diagnoseeinrichtung und eine Applikationseinrichtung umfasst. Die Diagnoseeinrichtung weist eine Bildaufnahmeeinrichtung zur Aufnahme wenigstens eines Bildes, einer Bildsequenz oder eines Videos des Gewebes, bspw. des zervikalen Gewebes auf. Das Bild, die Bildsequenz oder das Video wird an die Diagnoseeinrichtung übergeben, die zur Identifikation der Position und der Behandlungsbedürftigkeit verschiedener Abschnitte des Gewebes eingerichtet ist. Die Diagnoseeinrichtung kann dazu eingerichtet sein, pathologische Zustände des Gewebes zu erkennen und bezogen auf verschiedene Positionen und Behandlungsbedürftigkeiten verschiedener Zonen des Gewebes eine Behandlungsplankarte zu erstellen. Die Behandlungsplankarte stellt eine ortsabhängige Therapieempfehlung dar.

Die Applikationseinrichtung umfasst ein Instrument zur Applikation eines Behandlungsmediums auf Gewebe. Das Instrument kann beispielsweise ein Plasmainstrument, insbesondere ein Instrument zur Erzeugung eines Argonplasmastroms sein. Das Plasma ist vorzugsweise nicht im thermischen Gleichgewicht. Vorzugsweise weist es eine Gastemperatur (d.h. Temperatur der schweren Teilchen; z.B. Argonatome im Fall eines Argonplasmas) auf, die wesentlich niedriger ist als die Elektronentemperatur, so dass menschliches Gewebe wenigstens einen Zeitraum von einer oder wenigen Sekunden von dem Plasma berührt werden kann ohne eine schädigende Gewebekoagulation zu erfahren. Die Gastemperatur ist vorzugsweise so bemessen, dass die Gewebetemperatur zwar ansteigen kann, dabei jedoch unterhalb der Koagulationstemperatur von Proteinen (d.h. kleiner als 60°C) bleibt. Im Gegensatz zu einem Plasma mit höherer Gastemperatur, das zu einer thermischen Koagulation des Gewebes führt, wird solches Plasma als Kaltplasma oder warmes Plasma bezeichnet.

Die Applikationseinrichtung umfasst außerdem ein Gerät zur Speisung des Instruments. Das Gerät ist dazu eingerichtet, das Instrument mit Betriebsmedium und Energie zu versorgen. Das Betriebsmedium kann das Behandlungsmedium selbst oder ein Medium sein, aus dem unter zur Hilfenahme von Energie das Behandlungsmedium erzeugt wird. Ist das Behandlungsmedium kaltes oder warmes Argonplasma, ist das Betriebsmedium zum Beispiel Argon. Energie wird dann beispielsweise in Form von hochfrequenter Spannung und hochfrequentem Strom geliefert.

Zu der Applikationseinrichtung gehört außerdem eine Navigationseinrichtung, mittels derer Ort und Einwirkungsintensität des Behandlungsmediums auf das Gewebe steuerbar sind. Die Einwirkungsintensität kann beispielsweise durch die Stärke des Stroms, die Höhe der Spannung, die Form von Spannung und Strom, die Flussrate des Behandlungsmediums, zum Beispiel des Plasmastroms, und/oder durch die Dauer der Einwirkung des Behandlungsmediums auf eine bestimmte Stelle des Gewebes oder weitere Größen bestimmt sein.

Die Navigationseinrichtung kann dazu eingerichtet sein, dem Behandler die Behandlungsbedürftigkeit eines Gewebeabschnitts zu signalisieren, der aktuell von dem Behandlungsmedium erfasst wird, und somit eine Therapieempfehlung zu geben. Die Behandlungsbedürftigkeit bestimmt die Soll-Einwirkungsintensität, wobei beides in einem nicht linearen Zusammenhang stehen kann. Die Behandlungsbedürftigkeit wird durch eine Transformationsvorschrift aus der ortsbezogenen Diagnose, d.h. der Diagnosekarte abgeleitet. Die Transformationsvorschrift ist eine Erstellungsvorschrift für die Behandlungsplankarte und bildet ein Behandlungsmodell ab. Das Behandlungsmodell legt fest, zu welchem Grad einer Pathologie welche Behandlungsstärke oder Behandlungsintensität gehört.

Die Navigationseinrichtung kann die Behandlungsbedürftigkeit optisch, z.B. in Form der Behandlungsplankarte, akustisch, haptisch oder auf einem sonstigen Wege anzeigen. Darüber hinaus kann vorgesehen sein, dass die Navigationseinrichtung die Stärke oder Intensität des Behandlungsmediums beeinflusst, um es beispielsweise bei der Behandlung wenig behandlungsbedürftiger Bereiche abzuschwächen und bei der Einwirkung auf hochbehandlungsbedürftige Bereiche zu verstärken. Bei einem vom Anwender handgeführten Instrument kann dieser somit das Behandlungsmedium mit einer relativ gleichförmigen Geschwindigkeit über das Gewebe streichen, wobei die Navigationseinrichtung die Einwirkungsintensität ortsabhängig steuert. Ist die Möglichkeit der Modulation der Stärke des Behandlungsmediums hingegen eingeschränkt, wie es bei einem Kaltplasma als Behandlungsmedium der Fall sein kann, muss und kann die lokale Anpassung der Behandlungsstärke an die Behandlungsbedürftigkeit jedoch von dem Behandler durch die Führung des Instruments realisiert werden.

Weitere Ausbildungsformen der Navigationseinrichtung sind möglich. Beispielsweise kann die Navigationseinrichtung dem Behandler die zu behandelnden Bereiche optisch anzeigen und die erfolgte Behandlung der behandlungsbedürftigen Bereiche registrieren, sowie ebenfalls zur Anzeige bringen. Dazu kann die Behandlungsplankarte mit einer Einwirkungskarte überlagert werden, in der der Ort und die Stärke der vorgenommenen Behandlung vermerkt sind. Durch Überlagerung der Behandlungsplankarte mit der Einwirkungskarte kann eine Resultatkarte erzeugt werden, die lediglich die noch zu behandelnden Bereiche des Gewebes enthält. Optional können in der Resultatkarte auch überbehandelte Bereiche enthalten sein.

In einer weiteren Ausführungsform können Eingabemittel vorgesehen sein, die es dem behandelnden Arzt ermöglichen, die vorgeschlagene Behandlungsplankarte manuell zu verändern, in dem er Bereiche in die Behandlungsplankarte aufnimmt, die durch die Navigationseinrichtung nicht zur Behandlung vorgeschlagen worden sind. Dementsprechend kann er auch Bereiche aus der Behandlungsplankarte entfernen. Ebenso kann der Arzt die vorgeschlagenen Dosierungen bereichsweise nach oben oder unten anpassen.

Die zur Behandlungseinrichtung gehörige Bildaufnahmeeinrichtung kann zur Aufnahme einer Sequenz von Bildern eines bestimmten Gewebeabschnitts zu unterschiedlichen Zeitpunkten eingerichtet sein. Die verschiedenen Zeitpunkte können im Rahmen einer Videoaufnahme kurz hintereinander aufgenommene Frames sein, die eine Videosequenz bilden. Außerdem kann die Bildaufnahmeeinrichtung dazu genutzt werden, den betreffenden Gewebeabschnitt zu unterschiedlichen Untersuchungszeitpunkten beispielsweise im Abstand von Tagen oder Wochen aufzunehmen und Einzelbilder oder Bildsequenzen oder Videoaufnahmen zu machen.

In einer einfachen Ausführungsform ist die Diagnoseeinrichtung darauf eingerichtet, anhand der in einem Anfärbeversuch erzeugten spezifischen Reaktion auf die Färbung jedes von dem Bild erfassten Punkts des Gewebes den pathologischen Zustand des Gewebes zu ermitteln. Die Färbung kann beispielsweise bei einem Anfärbeversuch ermittelt werden, bei dem das Gewebe mit Lugolscher Lösung, Essigsäurelösung oder einem anderen Färbemittel, wie beispielsweise einem auf die Anwesenheit von HPV-Viren reagierenden Agens beaufschlagt wird. Es ist jedoch auch möglich, anstelle eines einzelnen Bilds dazu mehrere Bilder zu verwenden. Diese können beispielsweise zu unterschiedlichen Untersuchungszeitpunkten im Abstand von Tagen oder Wochen aufgenommen sein, woraus sich der Behandlungsfortschritt ermitteln lässt. Andere, z.B. optische Techniken, wie optische Emissionsspektroskopie (OES), optische Kohärenztomografie (OCT), Raman-Spektroskopie oder hyperspektrale bzw. multispektrale Kameraeinrichtungen sind zu diesem Zweck ebenfalls einsetzbar.

Es ist auch möglich, mehrere Bilder in kurzem zeitlichem Abstand als Bildsequenz, beispielsweise als Video aufzunehmen. Die Diagnoseeinrichtung kann darauf eingerichtet sein, daraus die Änderung der Färbungen in den Bildern, beispielsweise das Abklingverhalten einer Färbung, zu erfassen und anhand des Zeitverlaufs der Änderung der Färbungen jedem von den Bildern erfassten Punkt des Gewebes eine Kennzahl zuzuordnen, die die lokale Pathologie des Gewebes kennzeichnet. So entsteht zunächst ein Datensatz, der als Diagnosekarte bezeichnet werden kann. Die Navigationseinrichtung kann weiter dazu eingerichtet sein, aus den einzelnen Punkten der Diagnosekarte mittels der Transformationsvorschrift die erforderliche Einwirkungsstärke festzulegen und so eine Behandlungsplankarte zu erzeugen.

Die Behandlungsplankarte kann beispielsweise zur maschinellen Führung des Instruments genutzt werden. Sie dient dann zur Steuerung eines Positionierungssystems, z.B. eines Operationsroboters, der das Instrument führt. Alternativ kann das Instrument durch einen Behandler von Hand geführt sein. Dazu kann die Behandlungsplankarte auf einer Anzeigeeinrichtung, z.B. einem Display oder in einem Ausdruck angezeigt werden, um dem Behandler die Behandlungsbedürftigkeit einzelner Gewebepartien zu signalisieren. Außerdem ist es möglich, die Behandlungsplankarte als Augmented Reality der Ansicht des Gewebes zu überlagern, um die Behandlungsorte und Intensitäten sichtbar zu machen. Dazu kann die Behandlungsplankarte beispielsweise einem Kamerabild des zu behandelnden zervikalen Gewebes überlagert, in eine Brille des Behandlers eingespielt oder auf andere Weise sichtbar gemacht werden, beispielsweise indem die Behandlungsplankarte mittels eines Projektors auf das Gewebe projiziert wird. Sowohl die Brille als auch der Projektor bilden in diesen Fällen die genannte Anzeigeeinrichtung.

Weiter ist es vorteilhaft, wenn die Navigationseinrichtung eine Aufzeichnungseinrichtung umfasst, die dazu eingerichtet ist, die durch die Einwirkung des Behandlungsmediums auf das Gewebe erzielte Behandlungsdosis zu erfassen. Eine solche Erfassung der Behandlungsdosis kann beispielsweise erzielt werden, in dem Ort und Einwirkungsdauer des Behandlungsmediums auf das Gewebe, zum Beispiel mit einer Kamera erfasst und gegebenenfalls unter Berücksichtigung der Stärke des Behandlungsmediums, das heißt des Plasmastroms, die applizierte Behandlungsdosis ortsabhängig ermittelt wird. Die Navigationseinrichtung kann dazu eingerichtet sein, auf diese Weise eine Einwirkungskarte zu erstellen. Es ist möglich, die ortsabhängig erzielte Behandlungsdosis, d.h. die Einwirkungskarte mittels der Anzeigeeinrichtung sichtbar zu machen. Dazu kann die Anzeigeeinrichtung ein Display, eine VR-Brille oder eine sonstige Einrichtung aufweisen, die dem Behandler anzeigt, welche Dosis er bereits an welcher Stelle appliziert hat.

Dabei ist es insbesondere möglich, die Navigationseinrichtung dahingehend weiterzubilden, dass diese die Behandlungsplankarte mit der Einwirkungskarte überlagert und so eine Resultatkarte erstellt. In der Resultatkarte können Stellen, an denen die erzielte Behandlungsdosis die sich aus der Behandlungsplankarte ergebende lokale erforderliche Dosis erreicht, markiert sein. Die Markierung kann darin besten, dass in den ausreichend behandelten Bereichen die in der Behandlungsplankarte visualisierte Behandlungsbedürftigkeit getilgt wird. Der Behandler kann so erkennen, dass an dieser Stelle keine weitere Behandlung erforderlich oder eventuell sogar abträglich ist. So ist die Resultatkarte nach ausreichender Behandlung leer. Weiter kann die Navigationseinrichtung dazu ausgelegt sein, Stellen an denen Überbehandlung droht, das heißt die erzielte Dosis über der erforderlichen Dosis liegt, als Gefahrenzone beispielsweise farblich zu markieren, den Strom des Behandlungsmediums abzuschwächen oder zu sperren oder anderweitige Warnsignale, zum Beispiel akustisch, haptisch oder optisch wahrnehmbare Signale zu erzeugen. Zusammengefasst heißt dies, dass die Navigationseinrichtung dazu eingerichtet sein kann, die erzielte Behandlungsdosis in einer Einwirkungskarte darzustellen. Sie kann weiter dazu ausgelegt sein, eine Überlagerung der Behandlungsplankarte mit der Einwirkungskarte darzustellen, um noch verbleibende behandlungsbedürftige Bereiche für den Behandler als Resultatkarte optisch sichtbar zu machen.

Die Diagnoseeinrichtung kann mit einem Datenspeicher verbunden sein, der dazu eingerichtet ist, die Diagnose eines Patienten oder mehrerer Patienten spezifisch abzuspeichern. Insbesondere kann die Diagnoseeinrichtung dazu ausgerichtet sein, anhand eines einzelnen Bildes oder einer Bildsequenz, die während einer Untersuchung, beispielsweise während eines Anfärbeversuchs, aufgenommen worden ist, eine Diagnosekarte zu erstellen. Die Diagnoseeinrichtung und/oder die Navigationseinrichtung können dazu eingerichtet sein, auf Basis der Diagnosekarte eine Behandlungsplankarte zu erstellen. Die Diagnosekarte und/oder die Behandlungsplankarte, können in einem Speicher patientenspezifisch und zum Beispiel nach Untersuchungsterminen geordnet bereitgehalten werden. Weiter kann in dem Speicher die Transformationsvorschrift (d.h. die Erstellungsvorschrift) abgespeichert sein, auf deren Basis aus der Diagnosekarte die Behandlungsplankarte erstellt wird.

Weiter kann die Navigationseinrichtung dazu vorgesehen sein, die Diagnosekarte und/oder die Behandlungsplankarten in dem Speicher abzulegen und den Behandlungsterminen des Patienten und oder weiteren Daten zuzuordnen. Weitere Daten können patientenspezifischer und/oder behandlerspezifischer Natur sein. Solche Daten sind z.B. das Alter des Patienten, der körperliche Gesamtzustand des Patienten, weitere Erkrankungen des Patienten, Risikofaktoren des Patienten usw.

Die Navigationseinrichtung kann außerdem dazu eingerichtet sein, eine Einwirkungskarte zu erstellen, die die applizierte ortsabhängige Dosis des Behandlungsmediums wiedergibt. Die Navigationseinrichtung kann dazu eingerichtet sein, die Einwirkungskarte mit den aufgezeichneten Einwirkungsstärken an das Speichermedium zu übertragen. Das Speichermedium ist vorzugsweise darauf eingerichtet, die Einwirkungskarte patientenspezifisch und den Behandlungsterminen zugeordnet abzuspeichern.

Bei einer besonders bevorzugten Ausführungsform ist der Datenspeicher mit einem Behandlungsmonitor verbunden. Dieser ist dazu eingerichtet, die Diagnosekarte einer späteren Untersuchung mit der Diagnosekarte einer früheren Untersuchung zu vergleichen, um einen Behandlungserfolg zu ermitteln.

Weiter kann der Behandlungsmonitor darauf eingerichtet sein, den Zusammenhang zwischen der Diagnosekarte und der Behandlungsplankarte zu korrigieren. Dazu kann beispielsweise die Transformationsvorschrift geändert werden. Im einfachsten Fall ist die Erstellungsvorschrift eine Zuordnung einzelner Punkte der Diagnosekarte zu Punkten der Behandlungsplankarte mit gleichen Koordinaten. Einem gesunden Punkt in der Diagnosekarte wird ein nicht behandlungsbedürftiger Punkt in der Behandlungsplankarte zugeordnet. Einem Punkt mit einer behandlungswürdigen Gewebeeigenschaft, bspw. einer intraepithelialen Neoplasie, in der Diagnosekarte wird ein Punkt mit einer gegebenen Einwirkungsstärke in der Behandlungsplankarte zugeordnet. Dies ist eine 1/0-Zuordnung. Bei einem verfeinerten Behandlungsmodell kann eine lineare Zuordnung zwischen Grad der Pathologie und der Einwirkungsstärke vorgenommen werden. Es kann auch eine nichtlineare Zuordnung vorgenommen werden, z.B. indem ab einem bestimmten Grad der Pathologie die Einwirkungsstärke unterproportional zunimmt, bspw. um Gewebeschäden zu vermeiden. Außerdem ist es möglich, bei der Festlegung der erforderlichen Einwirkungsstärke in der Behandlungsplankarte nicht nur den zugeordneten koordinatengleichen Punkt der Diagnosekarte, sondern auch Umgebungspunkte mit einzubeziehen.

Der Behandlungsmonitor kann dazu eingerichtet sein, aus dem Vergleich der Diagnosekarten zu unterschiedlichen Untersuchungsterminen und der Einbeziehung der Einwirkungskarten die Transformation sukzessive zu verbessern. Aus der Diagnosekarte einer ersten Untersuchung geht eine erste Behandlungsplankarte hervor. Der Anwender führt die Behandlung dann anhand der Behandlungsplankarte durch, wobei seine Behandlungsaktivität in der Einwirkungskarte erfasst wird. Das Ergebnis dieser Einwirkung zeigt die in der zweiten (nachfolgenden) Untersuchung aufgenommene Diagnosekarte. Im Idealfall ist diese Karte leer, das heißt, sie zeigt keine pathologischen Veränderungen, bspw. keine intraepithelialen Neoplasien. Sind nach wie vor pathologische Veränderungen wie bspw. intraepithelialen Neoplasien vorhanden, entsteht wiederum eine Behandlungsplankarte, auf deren Basis eine weitere Behandlung durchgeführt wird. Die in der zweiten oder auch in weiteren Untersuchungen angefertigten Diagnosekarten sind die Grundlage für einen maschinellen Lernprozess, im Rahmen dessen die Transformationsvorschrift patientenspezifisch oder allgemein verbesserbar ist.

Außerdem kann die Transformationsvorschrift behandlerspezifisch verbessert werden, denn es ist davon auszugehen, dass die Einwirkungskarte behandlerspezifisch von der Behandlungsplankarte abweicht. Die behandlerspezifischen individuellen Abweichungen sind typischerweise nicht rein stochastisch, sondern von der individuellen Bewegungsmotorik des Behandlers abhängig. Der maschinelle Lernalgorithmus bildet eine Regelschleife deren Regelstrecke den Behandler enthält. Die von ihm eingebrachte nichtstochastische Abweichung zwischen der Behandlungsplankarte und der Einwirkungskarte wird von dem maschinellen Lernalgorithmus im Rahmen eines Regelprozesses beseitigt.

Auch durch den Anwender über eine Eingabeeinrichtung manuell eingeführte Änderungen in der vorgeschlagenen Behandlungsplankarte können in diesem Sinne durch den Lernalgorithmus erfasst und bewertet werden, um sie bei der Erstellung der Transformationsvorschrift zu nutzen.

Die Navigationseinrichtung kann darauf eingerichtet sein, patientenbezogene und/oder behandlerspezifische Behandlungsmodelle (Transformationsvorschriften) zu erstellen und patientenspezifisch und/oder behandlerspezifisch zu nutzen. Auf diese Weise können patientenspezifische Gegebenheiten ebenso wie individuelle Gewohnheiten des Behandlers bei der manuellen Führung des Instruments, die sonst zu teilweise unpassenden Einwirkungen auf das Gewebe führen könnten, beseitigt werden. Durch die behandlerspezifischen Behandlungsmodelle (Transformationsvorschriften) entstehen behandlerspezifische Behandlungsplankarten, die den Neigungen der Behandler, beispielsweise bestimmte Bereiche vorzuziehen oder die sonst zur Über- oder Unterbehandlung neigen, ausgleichen.

In einer weiteren Ausführungsform sind mehrere erfindungsgemäße Vorrichtungen durch ein Netzwerk, bspw. ein Cloud-Netzwerk miteinander verbunden und können somit optimierte Transformationsvorschriften austauschen. Ferner können die gesammelten Behandlungsdaten der vernetzten Geräte als Grundlage für neuronale Netzwerke oder andere maschinelle Lernverfahren dienen, um weitere Optimierungen der Transformationsvorschriften zu erhalten. Weiter ist es möglich, besonders erfolgreiche Behandlungsmodelle in einem zentralen Speicher bereit und abrufbar zu halten. Z.B. können bei einem Behandler, der sehr viele gleichartige patholigische Veränderung von Gewebe behandelt, durch den Lernalgorithmus ein weitgehend optimiertes Behandlungsmodell erzeugt werden. Dieses Behandlungsmodell kann dann an Behandlungseinrichtungen übertragen und dort genutzt werden, auch wenn dort nur seltener gleichartige Behandlungen durch zu führen sind. Insoweit ermöglich die erfindungsgemäße Einrichtung einen Erfahrungstransfer von hoch spezialisierten Behandlern auf weniger spezialisierte Behandler, ohne dass diese dazu selbst einen Lernprozess durchlaufen müssten. Das System lernt für sie.

In der Zeichnung ist die Erfindung in unterschiedlichen Aspekten veranschaulicht. Es zeigen:
Figur 1 Die erfindungsgemäße Behandlungseinrichtung in Blockdarstellung,
Figur 2 eine Diagnosekarte mit einer zweidimensionalen Darstellung von behandlungsbedürftigen (pathologischen) Bereichen des zervikalen Gewebes,
Figur 3 eine Behandlungsplankarte mit einer zweidimensionalen Darstellung behandlungsbedürftiger Bereiche,
Figur 4 eine im Entstehen begriffene Einwirkungskarte mit einer Kennzeichnung bereits behandelter Bereiche,
Figur 5 eine im Entstehen begriffene Behandlungsresultatkarte mit zweidimensionaler Darstellung des verbleibenden behandlungsbedürftigen Bereichs,
Figur 6 den logischen Ablauf der Behandlung und der Behandlungsoptimierung als Flussbild und
Figur 7 einen Logikplan des Betriebs der Behandlungseinrichtung.

Die erfindungsgemäße Vorrichtung und das erfindungsgemäße Verfahren werden im Folgenden beispielhaft an der Behandlung von zervikalen intraepithelialen Neoplasien erläutert. Es lässt sich aber auch auf andere Organe, bspw. den Darm und/oder andere Körperbereiche anwenden.

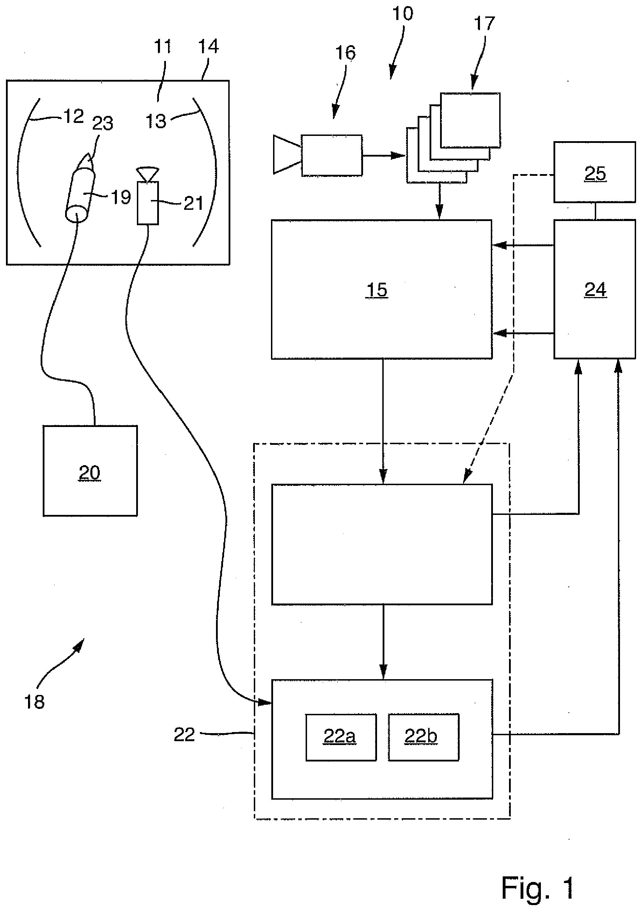

Figur 1 veranschaulicht eine erfindungsgemäße Behandlungseinrichtung 10 zur Behandlung von Cervixgewebe 11, dessen Zugang durch Lamellen 12, 13 eines Spekulums offengehalten wird. In einem durch eine Kamera aufgenommenen Bild 14 ist das Cervixgewebe 11 sichtbar. Bei entsprechender Bildgröße können auch die Lamellen 12, 13 in dem Bild 14 sichtbar sein.

Zu der Behandlungseinrichtung 10 gehört eine Diagnoseeinrichtung 15, die mit einer Bildaufnahmeeinrichtung 16, beispielsweise in Gestalt einer Kamera, kommunikativ verbunden ist. Die Bildaufnahmeeinrichtung 16 ist dazu eingerichtet, ein oder mehrere Bilder des Cervixgewebes 11 aufzunehmen und als Bild, als Folge von Einzelbildern oder als Videostream an die Diagnoseeinrichtung 15 zu übergeben.

Außerdem umfasst die Behandlungseinrichtung 10 eine Applikationseinrichtung 18, zu der ein Instrument 19 zur Einwirkung auf das Cervixgewebe 11, ein mit dem Instrument 19 verbundenes Gerät 20 zur Speisung des Instruments 19, eine Bildaufnahmeeinrichtung 21, zum Beispiel in Gestalt einer Kamera, und eine Navigationseinrichtung 22 gehören.

Das Instrument 19 dient der Applikation von Behandlungsmedium 23 auf das Gewebe 11. Das Behandlungsmedium 23 ist vorzugsweise ein Behandlungsmedium, das bei der Behandlung des Gewebes auf diesem keine unmittelbar sichtbaren Spuren hinterlässt, wie beispielsweise elektromagnetische Strahlung oder ein Plasma, jeweils mit einer Energie, die nicht zur sichtbaren Gewebekoagulation und somit Verfärbung führt. Als Plasma kommt insbesondere ein Nichtgleichgewichtsplasma, wie beispielsweise ein Kaltplasma oder ein anderes Plasma, in Frage, dessen Gas- und Ionentemperatur so niedrig ist, dass sie auch bei Einwirkung von einer oder wenigen Sekunden auf das Gewebe 11 nicht zu dessen unmittelbarer Devitalisierung durch Koagulation führt. Die Ionentemperatur des Plasmas liegt vorzugsweise unter 70 °C oder 60 °C noch weiter vorzugsweise unter 50 °C, im Idealfall unter 40 °C.

Das Gerät 20 speist das Instrument 19 mit den nötigen Betriebsmedien, beispielsweise Argon und elektromagnetischer Energie, wie beispielsweise HF-Strom zur Erzeugung eines Nichtgleichgewichtsplasmas, das als Behandlungsmedium 23 auf das Gewebe 11 trifft.

Das Instrument 19 ist vorzugsweise handgeführt und wird von dem Behandler genutzt, um das Behandlungsmedium 23 strichweise über das Gewebe 11 zu führen. Er kann dies unter Kontrolle mittels bloßen Auges oder unter Kontrolle mittels der Kamera 21 tun. Die Kamera 21 kann im Übrigen mit der Kamera 16 identisch oder als gesonderte Kamera ausgebildet sein. Das Instrument 19 kann allerdings auch ferngesteuert manuell oder maschinell geführt sein.

Der Behandler kann seine Tätigkeit, im Rahmen derer er das Gewebe 11 mit dem Behandlungsmedium 23 beaufschlagt, wie erwähnt, mit bloßem Auge, mittels der Kamera 21 auf einer Anzeigeeinrichtung, z.B. einem Bildschirm, oder über andere Mittel beispielsweise eine VR-Brille beobachten, in die ähnlich wie in das Bild eines Bildschirms Zusatzinformation eingeblendet werden kann. Diese Zusatzinformation wird von der Navigationseinrichtung 22 geliefert. Die Zusatzinformation kann insbesondere einen Therapievorschlag enthalten.

Die Behandlungseinrichtung 10 ist vorzugsweise mit einem Datenspeicher 24 verbunden, der in der Nähe des Behandlungsorts oder auch entfernt von diesem lediglich über ein datenübertragendes Netzwerk kommunizierend angeordnet ist. Der Datenspeicher 24 kann dazu eingerichtet sein, die von der Diagnoseeinrichtung 15 und von der Navigationseinrichtung 22 gelieferten Daten zu speichern und gegebenenfalls auch aufzubereiten. An den Datenspeicher 24 kann ein Behandlungsmonitor 25 angeschlossen sein, der dazu eingerichtet ist, den Erfolg der Behandlung zu bewerten und gegebenenfalls Daten zur Unterstützung der Bildanalyse zu liefern. Außerdem kann der Behandlungsmonitor auf die Navigationseinrichtung einwirken, um die vorzunehmende Behandlung zu beeinflussen. Der Behandlungsmonitor kann als Hard- und/oder Software realisiert sein, die die Behandlung überwacht.

Die nachfolgende Beschreibung der Funktionen der Diagnoseeinrichtung 15 und der Navigationseinrichtung 22 können auf einem geeigneten programmierbaren System wie einem oder mehreren Computern ablaufen, die zum Beispiel durch geeignete Programme dazu eingerichtet sind, die genannten Funktionen zu erbringen. Insoweit stellt die nachfolgende Funktionsbeschreibung auch eine Beschreibung der Beschaffenheit der Behandlungseinrichtung 10 dar.

Auf Basis der oben beschriebenen Grundstruktur der Behandlungseinrichtung 10 arbeitet diese wie folgt:

Dem eigentlichen Behandlungsschritt vorausgehend wird das Cervixgewebe 11 zunächst einer Untersuchung unterzogen, deren Ergebnis die Grundlage für die nachfolgende eigentliche Behandlung ist. Dazu wird auf das Cervixgewebe 11 zunächst eine geeignete Untersuchungsflüssigkeit wie beispielsweise Lugolsche Lösung oder Essigsäure oder dergleichen aufgetragen. Typischerweise werden zervikale intraepitheliale Neoplasien oder deren Vorstufen durch HPV-Viren hervorgerufen. Durch den HPV-Befall dysplastisches Gewebe wird in dem Anfärbeversuch sichtbar gemacht. Beispielsweise färbt sich pathologisch verändertes Gewebe dabei weiß, wobei die Weißfärbung mit der Zeit wieder abklingt.

Für die Behandlung von anderen Organen und/oder Körperbereichen können andere Techniken eingesetzt werden, um das zu behandelnde Gewebe für die Diagnoseeinrichtung 15 detektierbar zu machen.

Die Diagnoseeinrichtung 15 kann dazu eingerichtet sein, ein einzelnes Bild des gefärbten Gewebes 11 auszuwerten, um den Umriss (d.h. den Ort und die Form) des durch Weißfärbung erkannten befallenen Gebiets zu ermitteln. Auch kann die Diagnoseeinrichtung 15 dazu eingerichtet sein, die ortsspezifische Intensität der Weißfärbung zu erfassen. Alternativ oder zusätzlich kann die Diagnoseeinrichtung 15 dazu eingerichtet sein, mehrere Bilder 17 oder eine während des Anfärbeversuchs aufgenommene Videosequenz auszuwerten. Die Diagnoseeinrichtung kann dazu eingerichtet sein, aus dem Abklingverhalten, d.h. dem Schwinden der Farbe des angefärbten Bereichs, beispielsweise auf den Grad der Pathologie, d.h. zum Beispiel auf die Schwere des HPV-Befalls und/oder auf den Grad des Vorhandenseins intraepithelialer Neoplasien und somit die Behandlungsbedürftigkeit des Gewebes zu schließen. Die Diagnoseeinrichtung 15 kann dazu eingerichtet sein, die gewonnenen Daten zeit- und patientenspezifisch an den Datenspeicher 24 zu übertragen. Aus dem Datenspeicher 24 kann die Diagnoseeinrichtung 15 zum Beispiel Information über frühere Untersuchungen oder patiententypabhängiges Abklingverhalten, färbelösungstypische Gewebereaktionen und dergleichen erhalten. In Figur 1 ist dies durch Pfeile zwischen dem Datenspeicher 24 und der Diagnoseeinrichtung 15 symbolisiert.

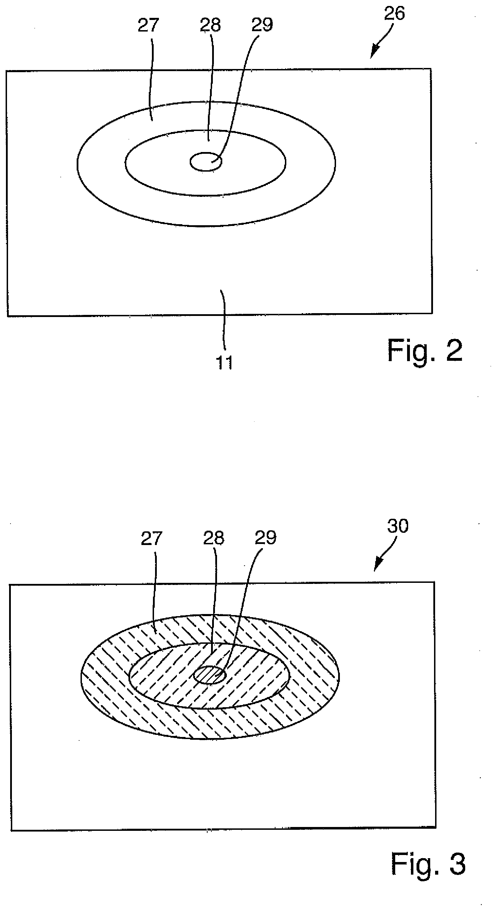

Die Diagnoseeinrichtung ist dazu eingerichtet, zunächst eine Diagnosekarte 26 zu erstellen, wie sie in Figur 2 veranschaulicht ist. Diese kann als zweidimensionale Abbildung des Cervixgewebes 11 angesehen werden, in der mindestens eine gegebenenfalls aber auch mehrere Zonen 27, 28, 29 markiert sind, die von HP-Viren befallenes Gewebe und/oder schon vorhandene zervikale intraepitheliale Neoplasien veranschaulichen. Die Zonen 27, 28, 29 können verschiedene Schweregrade des Befalls oder der Neoplasien veranschaulichen. Die Diagnosekarte 26 kann auf einem Monitor direkt zur Anzeige gebracht oder alternativ auch lediglich intern verarbeitet werden.

Die Diagnosekarte 26 ist die Grundlage für die Erstellung einer Behandlungsplankarte 30 wie sie in Figur 3 veranschaulicht ist. Die Behandlungsplankarte 30 zeigt für die Zonen 27, 28, 29 nun die Behandlungsbedürftigkeit an. Die Behandlungsplankarte 30 kann von der Navigationseinrichtung 22 allein auf Basis der Diagnosekarte 26 oder auch unter zusätzlichem Rückgriff auf Patientendaten erzeugt werden. Beispielsweise kann bei der Erstellung der Behandlungsplankarte 30 die Historie der betreffenden Patientin beispielsweise in Gestalt der Anzahl und Intensität der bereits durchlaufenen Behandlungen stehen. Auch Alter und der sonstige Gesundheitsstatus der Patientin können Berücksichtigung finden.

Die Erstellung der Behandlungsplankarte 30 aus zumindest der Diagnosekarte erfolgt mittels eines Behandlungsmodells, das eine Transformationsvorschrift ist. Im einfachsten Fall ordnet die Navigationseinrichtung 22 zur Erstellung der Behandlungsplankarte 30 den Zonen 27, 28, 29 eine Behandlungsbedürftigkeit proportional der Schwere der vorliegenden Läsion (HPV-Befall oder vorhandene Neoplasien) zu. Der durch die Transformationsvorschrift (d.h. das Behandlungsmodell) gegebene Zusammenhang kann allerdings auch nach einer nichtlinearen Funktion und wie oben erwähnt unter zusätzlicher Berücksichtigung der Behandlungshistorie erfolgen.

Bei einer bevorzugten Ausführungsform können Eingabemittel, z.B. in Gestalt einer Tastatur, einer Computermaus, eines Touchscreens, einer Sprachsteuerung usw., vorgesehen sein, mittels derer der Behandler die vorgeschlagene Behandlungsplankarte 30 manuell verändern kann. Dazu kann er Gewebebereiche in die Behandlungsplankarte aufnehmen, die durch die Diagnoseeinrichtung nicht zur Behandlung vorgeschlagen worden sind. Er kann auch Bereiche aus der Behandlungsplankarte entfernen. Ebenso kann der Behandler die vorgeschlagenen Dosierungen bereichsweise nach oben oder unten anpassen.

Die Behandlungsplankarte 30 wird dem Behandler auf einer geeigneten Wiedergabeeinrichtung, beispielsweise mittels eines Screens oder einer VR-Brille, wiedergegeben oder mittels eines Projektors direkt auf das Cervixgewebe 11 projiziert. Dies findet nach der Untersuchung während der eigentlichen Behandlung statt, bei der der Behandler mittels des Instruments 19 das Behandlungsmedium 23 auf das Gewebe 11 appliziert. Dieses wirkt auf das Gewebe 11 ein, was jedoch an dem Gewebe 11 nicht unmittelbar sichtbar wird. Die Navigationseinrichtung 22 ist aber dazu eingerichtet, den Ort, die Bewegung und die Einwirkungsdauer des Behandlungsmediums 23 über und auf das Gewebe 11 zu registrieren und zumindest bei nicht konstanter Stärke des Behandlungsmediums 23 auch dessen Stärke zu berücksichtigen. Optional kann die Navigationseinrichtung 22 mit dem Generator 20 kommunikativ verbunden sein, um dessen Betriebsdaten zu nutzen, um die Stärke des Behandlungsmediums 23 zu bestimmen und zu berücksichtigen oder auch um die Stärke des Behandlungsmediums 23 vorzugeben. Dazu kann die Navigationseinrichtung 22 eine Aufzeichnungseinrichtung 22a umfassen.

Aus der registrierten Spur der Einwirkung des Behandlungsmediums 23 auf das Gewebe 11 erstellt die Navigationseinrichtung 22 während der Behandlung eine Einwirkungskarte 31. Die Navigationseinrichtung 22 ist dazu eingerichtet, die Zone der Einwirkung 32 und gegebenenfalls die Zone stärkerer Einwirkung 33 den Zonen 27, 28, 29 zu überlagern. Die Einwirkungskarte 31 muss dem Behandler nicht zwingend angezeigt werden. Die Navigationseinrichtung kann aber dazu eingerichtet sein, aus ihr eine Resultatkarte 34 zu erstellen, in der diejenigen Teile der Zonen 27, 28, 29 getilgt sind, die bereits ausreichende Behandlung mit dem Behandlungsmedium 23 erfahren haben. Die Behandlungsplankarte 30, die Einwirkungskarte 31 und/oder die Resultatkarte 34 können mittels einer zu der Navigationseinrichtung 22 gehörigen Anzeigeeinrichtung 22b allein oder überlagert mit einem Livebild der Kamera 21 wiedergegeben werden. Die Anzeigeeinrichtung kann z.B. eine VR-Brille, ein Monitor oder ähnliches sein.

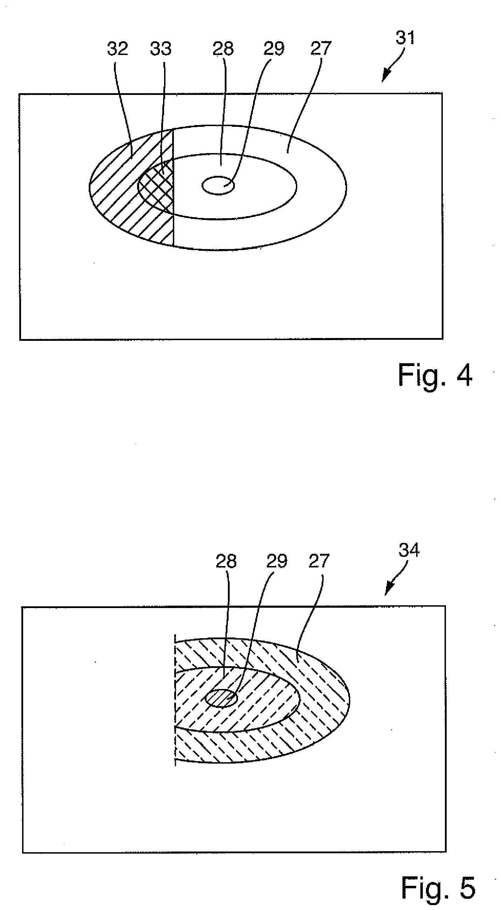

Die Resultatkarte 34 kann dem Behandler mit einem der oben genannten geeigneten Anzeigemedien (Projektor, Monitor, VR-Brille, usw.) zur Anzeige gebracht werden. Der Behandler wird somit das Instrument 23 so lange strichweise über die Zonen 27, 28, 29 führen, bis diese aus seinem Blickfeld verschwinden. Zu Beginn der Behandlung ist die dem Behandler angezeigte Karte mit der Behandlungsplankarte 30 gemäß Figur 3 identisch und stellt eine Therapieempfehlung dar. Während der Behandlung werden die behandelten Bereiche getilgt, wie es Figur 5 veranschaulicht. Zu Ende der Behandlung ist die Karte leer, es sind zum Zeichen der vollständigen Behandlung alle Zonen 27, 28, 29 getilgt. Die Resultatkarte 34 kann als Differenz aus der Behandlungsplankarte 30 und der Einwirkungskarte 31 gebildet werden. Wenn die Behandlungsplankarte 30 und die Einwirkungskarte 31 identisch sind, also eine genau planmäßige Behandlung erfolgt ist, ist die Resultatkarte 34 leer (d.h. überall Null). Bestehen Abweichungen zwischen der Einwirkungskarte 31 und der Behandlungsplankarte 30, sind diese in der Resultatkarte dokumentiert.

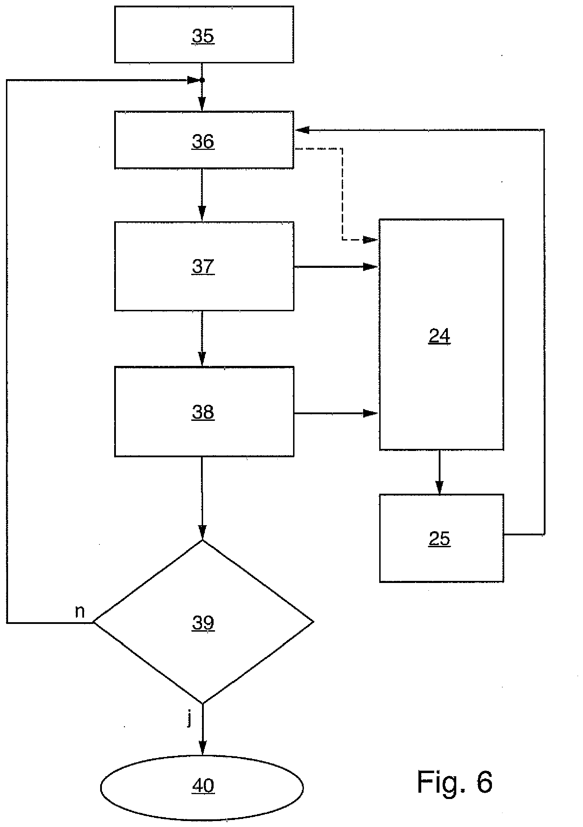

Figur 6 veranschaulicht den Betrieb der Behandlungseinrichtung 10 in logischen Blöcken als Ablaufplan. Der Ablauf beginnt in Block 35 mit der Untersuchung des Cervixgewebes 11 der Patientin wie vorstehend beschrieben. Die Diagnoseeinrichtung 15 erstellt aus dem mindestens einen aufgenommenen Bild 17 oder einer Bildsequenz in Block 36 zunächst die Diagnosekarte 26 und aus dieser unter Zuhilfenahme der Transformationsvorschrift (d.h. des Behandlungsmodells) die Behandlungsplankarte 30 nach Figur 3. Die Transformationsvorschrift ist in der Navigationseinrichtung 22 gespeichert und kann beispielsweise darin bestehen, die Einwirkungsstärke lokal an die Stärke der Läsion zum Beispiel proportional anzupassen. Alternativ zu dieser linearen Zuordnung zwischen Stärke der Läsion und Einwirkungsstärke kann auch ein nicht linearer Zusammenhang gewählt werden, beispielsweise um bei starken Läsionen eine Gewebeschädigung durch zu starke Einwirkungsstärke zu verhindern.

Anhand der Behandlungsplankarte 30 kann der Behandler nun in Block 37 die Behandlung gemäß der Behandlungsplankarte 30 durchführen. Damit endet die aktuelle Behandlung der Patientin zunächst. Die Behandlungsplankarte 30, die den Therapievorschlag repräsentiert, und/oder die Einwirkungskarte 31, die die tatsächlich vorgenommene Behandlung dokumentiert, können beide in dem Speicher 24 abgelegt sein. Zusätzlich kann das Behandlungsmodell in dem Speicher 24 abgelegt sein.

Zu einem späteren Zeitpunkt kann sich die Patientin erneut zur Untersuchung vorstellen, wie Block 38 symbolisiert. Dabei wird mittels der Diagnoseeinrichtung 15 wiederum der durchgeführte Anfärbeversuch dokumentiert und ausgewertet und eine Diagnosekarte 26' erstellt.

Die von Block 37 an den Datenspeicher 24 übergebene Einwirkungskarte 31 (und/oder die Resultatkarte 34) und die in der neuen Untersuchung in Block 38 von der Diagnoseeinrichtung 15 erstellte Diagnosekarte 26' können in dem Speicher 24 abgelegt sein. Durch Vergleich der ersten Diagnosekarte 26, der Behandlungsplankarte 30, der Einwirkungskarte 31 und der in Schritt 38 gewonnenen Diagnosekarte 26' kann der Behandlungsmonitor 25 ermitteln ob die von der Diagnosekarte 26 zur Behandlungsplankarte 30 führende Transformationsvorschrift, d.h. das Behandlungsmodell, verbessert werden kann oder muss:

Beispielsweise kann deutlich werden, dass die Zone 27 und die Zone 29 ausreichend behandelt sind, während in der Zone 28 weiterhin behandlungsbedürftige Bereiche existieren. Der Behandlungsmonitor 25 kann dazu eingerichtet sein, dies festzustellen und eine Korrekturempfehlung für die Transformationsvorschrift von der Diagnosekarte 26' in die Behandlungsplankarte zu liefern. In dem genannten Beispielfall wird die Transformationsvorschrift (das Behandlungsmodell) für Läsionen des Maßes der Zone 28 dahingehend geändert, dass eine erhöhte Einwirkungsintensität festgelegt wird. Die so gewonnene Änderung der Transformationsvorschrift kann für die konkrete Patientin oder auch ein größeres Patientenkollektiv, zum Beispiel Patientinnen gleichen oder ähnlichen Alters, gleicher Historie (Anzahl der Kinder, allgemeiner körperlicher Status, Gewicht, Konstitution usw.) festgelegt werden.

Wird in Block 38 und 39 vollständiger Behandlungserfolg festgestellt, endet das Verfahren in Block 40. Falls nicht, wird mit dem Verfahren mit Block 36 fortgesetzt.

Der Ablauf nach Figur 6 bezieht die Funktion der Behandlungseinrichtung 10 und somit den Behandler ein. Nachdem in Block 37 die Behandlung gemäß Behandlungsplankarte 30 von einem Behandler, d.h. einer menschlichen Person durchgeführt wird, ist hier mit Ungenauigkeiten zu rechnen. Beispielsweise können Behandler dazu neigen, die Zonen 27, 28, 29 zu langsam, zu schnell, überschießend oder unzureichend zu behandeln. Dies resultiert dann bei der zweiten Untersuchung in Block 38 in noch vorhandenen Läsionen, die von dem Behandlungsmonitor 25 erkannt und in eine Korrektur der Transformationsvorschrift umgesetzt werden können. Die Behandlungseinrichtung 10 kann deswegen dazu eingerichtet sein, behandlerspezifische Transformationsvorschriften (Behandlungsmodelle) zu ermitteln und vorzuhalten und anzuwenden, sodass jeder Behandler ungeachtet seiner persönlichen Fertigkeit, ein wünschenswertes Behandlungsergebnis erzielt. Es ist aber auch möglich, anhand der Arbeit hoch spezialisierter und sehr erfolgreicher Behandlern erzeugte Behandlungsmodelle zur Nutzung durch weniger hoch spezialisierte Behandler bereit zu stellen. So können Behandlungsmodelle von Spezialkliniken an weniger spezialisierte Kliniken übertragen werden und auch dort zu guten Behandlungserfolgen führen.

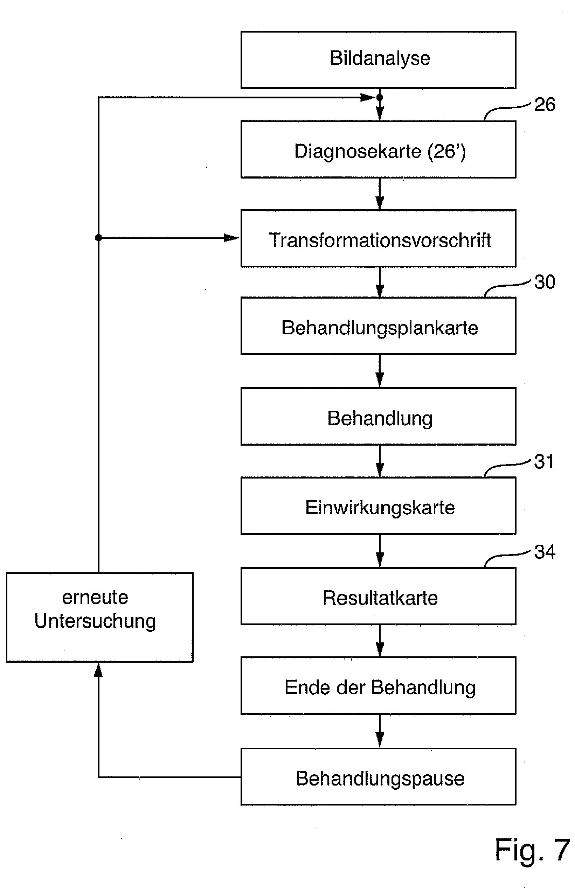

Zur weiteren Verdeutlichung des erfindungsgemäßen Optimierungsverfahrens zur Verbesserung der Diagnose und Behandlungsqualität von zervikalen intraepithelialen Neoplasien wird auf Figur 7 verwiesen. Am Anfang steht die Untersuchung einer Patientin mittels Bildanalyse. Die Diagnoseeinrichtung 15 ist dazu eingerichtet, die aufgenommenen Bilder zu analysieren und daraus eine Diagnosekarte 26 zu erstellen. Die Diagnosekarte 26 wird mittels einer Transformationsvorschrift (Behandlungsmodell) in die Behandlungsplankarte 30 übersetzt. Es folgt die manuelle oder maschinelle Behandlung des Gewebes 11 anhand der Behandlungsplankarte 30 und daraufhin unter Registrierung der durchgeführten Behandlung die Erstellung einer Einwirkungskarte 31, die die erfolgte Behandlung (Einwirkung) dokumentiert. Die Einwirkungskarte 31 ändert sich dynamisch während der Einwirkung. Ist die Behandlung beendet, enthält die Einwirkungskarte 31 die lokal differenzierten Einwirkungen. Nur im Idealfall sind diese mit den Vorgaben der Behandlungsplankarte 30 identisch und die Resultatkarte 34 somit leer.

Damit ist die Behandlung zunächst beendet und es erfolgt typischerweise eine mehrtägige bis mehrwöchige Behandlungspause. In einer erneuten Untersuchung, wiederum mit Bildanalyse durch die Diagnoseeinrichtung 15, wird eine neue Diagnosekarte 26' und gegebenenfalls auch eine Korrektur der Transformationsvorschrift (des Behandlungsmodells) ermittelt, wonach die vorgenannten Blöcke erneut durchlaufen werden. Durch die genannte Anpassung der Transformationsvorschrift werden einerseits typische behandlerspezifische Abweichungen zwischen der Behandlungsplankarte 30 und der Einwirkungskarte 31 minimiert und andererseits werden patientenspezifisch unterschiedliche Anforderungen an die Behandlungsintensität berücksichtigt. Dies erleichtert die Optimierung des Therapievorschlags in Gestalt der Behandlungsplankarte 30.

Die erfindungsgemäßen Vorrichtungen in verschiedenen Einrichtungen können über ein Netzwerk miteinander verbunden sein und untereinander angepasste Transformationsvorschriften zur patiententypischen Behandlung austauschen. Außerdem kann der größere Datensatz der vernetzten Vorrichtungen zur besseren Anpassung der Transformationsvorschriften im Zuge von maschinellen Lernverfahren eingesetzt werden.

Die erfindungsgemäße Behandlungseinrichtung ermöglicht eine Behandlung von Gewebe mit einem keine unmittelbare Gewebespur hinterlassenden Behandlungsmedium (23) sowohl mit manueller als auch mit maschineller Instrumentenführung und unter Anpassung der Behandlungsplankarte aufgrund vorheriger Behandlungen. Die Behandlungsplankarte stellt die Behandlungsvorschrift für den Behandler dar. Das erfindungsgemäße Konzept gestattet die Anpassung an verschiedene Patienten oder Patientenkollektive sowie auch die Individualisierung der Erstellung der Behandlungsplankarte hinsichtlich der nutzenden Behandler.

### Bezugszeichen:

- 10: Behandlungseinrichtung
- 11: Cervixgewebe
- 12, 13: Lamellen
- 14: Bild
- 15: Diagnoseeinrichtung
- 16: Bildaufnahmeeinrichtung
- 17: Bilder, Video
- 18: Applikationseinrichtung
- 19: Instrument
- 20: Gerät zur Speisung des Instruments 19
- 21: Bildaufnahmeeinrichtung / Kamera
- 22: Navigationseinrichtung
- 22a: Aufzeichnungseinrichtung
- 22b: Anzeigeeinrichtung
- 23: Behandlungsmedium
- 24: Datenspeicher
- 25: Behandlungsmonitor
- 26: Diagnosekarte
- 26': Diagnosekarte bei zweiter oder weiterer Untersuchung
- 27 - 29: Zonen in der Diagnosekarte 26
- 30: Behandlungsplankarte
- 31: Einwirkungskarte
- 32, 33: Zonen verschieden starker Einwirkung
- 34: Resultatkarte
- 35 - 40: Blöcke

## Patentansprüche

1. Behandlungseinrichtung (10) zur Behandlung von menschlichem oder tierischem Gewebe, mit:
einer Diagnoseeinrichtung (15) mit einer Bildaufnahmeeinrichtung (16) zur Aufnahme wenigstens eines Bildes (17) des Gewebes (11), wobei die Diagnoseeinrichtung (15) zur Identifikation von behandlungsbedürftigen Zonen (27, 28, 29) eingerichtet ist,
einer Applikationseinrichtung (18), zu der:
- ein Instrument (19) zur Applikation eines Behandlungsmediums (23) auf Gewebe (11) einer zu behandelnden Person,
- ein Gerät (20), das mit dem Instrument (19) verbindbar ist, um dieses mit Betriebsmedium und Energie zu versorgen, sowie
- eine Navigationseinrichtung (22) gehören, mittels derer Ort und Soll-Einwirkungsintensität des Behandlungsmediums (23) auf das Gewebe (11) bestimmbar sind.

2. Behandlungseinrichtung nach Anspruch 1, **dadurch gekennzeichnet, dass** die Diagnoseeinrichtung (15) dazu eingerichtet ist, in jedem von der Bildaufnahmeeinrichtung (16) aufgenommenen Bild (17) Gebiete (27, 28, 29) unterschiedlicher Läsion zu identifizieren und so eine Diagnosekarte (26) zu generieren.

3. Behandlungseinrichtung nach Anspruch 2, **dadurch gekennzeichnet, dass** die Navigationseinrichtung (22) und/oder die Diagnoseeinrichtung (15) darauf eingerichtet ist, aus der Diagnosekarte (26) jedem von dem Bild (17) erfassten Punkt des Gewebes (11) eine Einwirkungsstärke zuzuordnen und so eine Behandlungsplankarte (30) zu erzeugen.

4. Behandlungseinrichtung nach Anspruch 3, **dadurch gekennzeichnet, dass** die Navigationseinrichtung (22) und/oder die Diagnoseeinrichtung (15) darauf eingerichtet ist, zur Erzeugung der Behandlungsplankarte (30) aus der Diagnosekarte (26) eine Transformationsvorschrift anzuwenden.

5. Behandlungseinrichtung nach Anspruch 4, **dadurch gekennzeichnet, dass** die Navigationseinrichtung (22) und/oder die Diagnoseeinrichtung (15) darauf eingerichtet ist, zeitliche Änderungen der bei dem Anfärbeversuch erzeugten Färbungen in den Bildern (17) zu erfassen und anhand der Änderungen jedem von den Bildern (17) erfassten Punkt des Gewebes (11) eine erforderliche Einwirkungsstärke zuzuordnen.

6. Behandlungseinrichtung nach einem der vorstehenden Ansprüche, **dadurch gekennzeichnet, dass** das Behandlungsmedium (23) ein nichtthermisches Plasma ist.

7. Behandlungseinrichtung nach Anspruch 3, 4 oder 5, **dadurch gekennzeichnet, dass** die Navigationseinrichtung (22) eine Anzeigeeinrichtung (22b) aufweist, die dazu eingerichtet ist, die Behandlungsplankarte (30) optisch sichtbar in Bezug auf das zu behandelnde Gewebe (11) darzustellen.

8. Behandlungseinrichtung nach Anspruch 7, **dadurch gekennzeichnet, dass** die Navigationseinrichtung (22) eine Beobachtungseinrichtung (21), die zur visuellen Inspektion des zu behandelnden Gewebes durch einen Behandler eingerichtet ist, und eine Anzeigeeinrichtung (22b) aufweist, die dazu eingerichtet ist, die Behandlungsplankarte (30) für den Behandler optisch sichtbar dem Gewebe (11) zu überlagern.

9. Behandlungseinrichtung nach einem der vorstehenden Ansprüche, **dadurch gekennzeichnet, dass** die Navigationseinrichtung (22) eine Aufzeichnungseinrichtung (22a) umfasst, mittels derer die durch die Einwirkung des Behandlungsmediums (23) auf das Gewebe (11) erzielte Behandlungsdosis und/oder der Einwirkungsort erfassbar ist.

10. Behandlungseinrichtung nach Anspruch 6, 7 und 9 oder alternativ 7, 9 und 10, **dadurch gekennzeichnet, dass** die Anzeigeeinrichtung (22b) darauf eingerichtet ist, die erzielte Behandlungsdosis in einer Einwirkungskarte (31) darzustellen.

11. Behandlungseinrichtung nach einem der vorstehenden Ansprüche, **dadurch gekennzeichnet, dass** die Diagnoseeinrichtung (15) mit einem Datenspeicher (24) verbunden ist, um die Diagnosekarte (26) patientenspezifisch abzuspeichern.

12. Behandlungseinrichtung nach Anspruch 11, **dadurch gekennzeichnet, dass** die Diagnoseeinrichtung (15) und/oder die Navigationseinrichtung (22) dazu eingerichtet ist, eine Einwirkungskarte (30) zu erstellen und mit dem Datenspeicher (24) verbunden ist, um die Einwirkungskarte (30) patientenspezifisch abzuspeichern.

13. Behandlungseinrichtung nach Anspruch 11 oder 12, **dadurch gekennzeichnet, dass** die Diagnoseeinrichtung (15) und/oder die Navigationseinrichtung (22) dazu eingerichtet ist, eine Einwirkungskarte (30) zu erstellen und mit dem Datenspeicher (24) verbunden ist, um die Einwirkungskarte (30) behandlerspezifisch abzuspeichern.

14. Behandlungseinrichtung nach Anspruch 12 oder 13, **dadurch gekennzeichnet, dass** der Datenspeicher (24) mit einem Behandlungsmonitor (25) verbunden ist, der dazu eingerichtet ist, anhand einer zu einem ersten Untersuchungstermin aufgenommenen Behandlungsplankarte (30), der Einwirkungskarte (31) und einer zu einem zweiten Untersuchungstermin aufgenommenen Behandlungsplankarte (30) den Behandlungserfolg zu ermitteln.

15. Verfahren zur Erstellung einer Behandlungsplankarte (30) zur Behandlung von menschlichem oder tierischem Gewebe, bei dem:
in einem ersten Untersuchungsschritt eine erste flächige Untersuchung des Gewebes (11) vorgenommen und anhand der Untersuchung unter Nutzung einer Erstellungsvorschrift eine erste Behandlungsplankarte (30) erstellt und gespeichert wird,
in einem ersten Behandlungsschritt eine erste Behandlung entsprechend der Behandlungsplankarte (30) durchgeführt wird,
in einem zweiten Schritt eine zweite flächige Untersuchung des Gewebes vorgenommen und anhand der Untersuchung unter Nutzung der Erstellungsvorschrift eine zweite Behandlungsplankarte (30) erstellt und gespeichert wird,
aus der ersten und der zweiten Behandlungsplankarte (30) eine Korrekturempfehlung für die Erstellungsvorschrift ermittelt wird.
